# EUROPEAN PATENT APPLICATION

(11) **EP 1 719 817 A1**
(43) Date of publication of application: **08.11.2006**
(21) Application number: 05709922.8
(22) Date of filing: 02.02.2005
(51) Int. Cl.: C12N 15/12, C07K 14/47

(54) **CARCINOSTATIC METHOD USING BRCA1-BARD1 PATHWAY**

(30) Priority: 02.02.2004 US 541287 P
(71) Applicant: Locomogene, Inc., Tokyo 105-0001 (JP); St. Marianna University School of Medicine, Kawasaki-shi, Kanagawa 216-8511 (JP)
(72) Inventor: OHTA, Tomohiko, Setagaya-ku, Tokyo 1550032 (JP)
(74) Representative: Woods, Geoffrey Corlett
(86) International application number: PCT/JP2005/001870
(87) International publication number: WO 2005/073379

(57) **Abstract**

The present invention provides a method of polyubiquitinating a nucleophosmin comprised of reacting the nucleophosmin *in vitro* or *in vivo* with BRCA1-BARD1. The present invention also provides a method of inhibiting polyubiquitination of nucleophosmin comprised of phosphorylating BARD1 using CDK2-cyclin E and/or CDK2-cyclin A.

SEQUENCE LISTING

<110> Locomogene, Inc.,
      St. Marianna University School of Medicine
      Ohta, Tomohiko

<120> CARCINOSTATIC METHOD USING BRCA1-BARD1 PATHWAY

<130> PCT05-0001

<150> US60/541287
<151> 2004-02-02

<160> 24

<170> PatentIn version 3.2

<210> 1
<211> 1333
<212> DNA
<213> Homo sapiens


<220>
<221> CDS
<222> (101)..(985)

<400> 1
ggggccctgg tgtgattccg tcctgcgcgg ttgttctctg gagcagcgtt cttttatctc      60

cgtccgcctt ctctcctacc taagtgcgtg ccgccacccg atg gaa gat tcg atg      115
                                            Met Glu Asp Ser Met
                                            1               5

gac atg gac atg agc ccc ctg agg ccc cag aac tat ctt ttc ggt tgt      163
Asp Met Asp Met Ser Pro Leu Arg Pro Gln Asn Tyr Leu Phe Gly Cys
                10                  15                  20

gaa cta aag gcc gac aaa gat tat cac ttt aag gtg gat aat gat gaa      211
Glu Leu Lys Ala Asp Lys Asp Tyr His Phe Lys Val Asp Asn Asp Glu
            25                  30                  35

aat gag cac cag tta tct tta aga acg gtc agt tta ggg gct ggt gca      259
Asn Glu His Gln Leu Ser Leu Arg Thr Val Ser Leu Gly Ala Gly Ala
        40                  45                  50

aag gat gag ttg cac att gtt gaa gca gag gca atg aat tac gaa ggc      307
Lys Asp Glu Leu His Ile Val Glu Ala Glu Ala Met Asn Tyr Glu Gly
    55                  60                  65

agt cca att aaa gta aca ctg gca act ttg aaa atg tct gta cag cca      355

Ser Pro Ile Lys Val Thr Leu Ala Thr Leu Lys Met Ser Val Gln Pro
70                  75                  80                  85

acg gtt tcc ctt ggg ggc ttt gaa ata aca cca cca gtg gtc tta agg        403
Thr Val Ser Leu Gly Gly Phe Glu Ile Thr Pro Pro Val Val Leu Arg
                90                  95                  100

ttg aag tgt ggt tca ggg cca gtg cat att agt gga cag cac tta gta        451
Leu Lys Cys Gly Ser Gly Pro Val His Ile Ser Gly Gln His Leu Val
            105                 110                 115

gct gtg gag gaa gat gca gag tca gaa gat gaa gag gag gag gat gtg        499
Ala Val Glu Glu Asp Ala Glu Ser Glu Asp Glu Glu Glu Glu Asp Val
        120                 125                 130

aaa ctc tta agt ata tct gga aag cgg tct gcc cct gga ggt ggt agc        547
Lys Leu Leu Ser Ile Ser Gly Lys Arg Ser Ala Pro Gly Gly Gly Ser
    135                 140                 145

aag gtt cca cag aaa aaa gta aaa ctt gct gct gat gaa gat gat gac        595
Lys Val Pro Gln Lys Lys Val Lys Leu Ala Ala Asp Glu Asp Asp Asp
150                 155                 160                 165

gat gat gat gaa gag gat gat gat gaa gat gat gat gat gat gat ttt        643
Asp Asp Asp Glu Glu Asp Asp Asp Glu Asp Asp Asp Asp Asp Asp Phe
                170                 175                 180

gat gat gag gaa gct gaa gaa aaa gcg cca gtg aag aaa tct ata cga        691
Asp Asp Glu Glu Ala Glu Glu Lys Ala Pro Val Lys Lys Ser Ile Arg
            185                 190                 195

gat act cca gcc aaa aat gca caa aag tca aat cag aat gga aaa gac        739
Asp Thr Pro Ala Lys Asn Ala Gln Lys Ser Asn Gln Asn Gly Lys Asp
        200                 205                 210

tca aaa cca tca tca aca cca aga tca aaa gga caa gaa tcc ttc aag        787
Ser Lys Pro Ser Ser Thr Pro Arg Ser Lys Gly Gln Glu Ser Phe Lys
    215                 220                 225

aaa cag gaa aaa act cct aaa aca cca aaa gga cct agt tct gta gaa        835
Lys Gln Glu Lys Thr Pro Lys Thr Pro Lys Gly Pro Ser Ser Val Glu
230                 235                 240                 245

gac att aaa gca aaa atg caa gca agt ata gaa aaa ggt ggt tct ctt        883
Asp Ile Lys Ala Lys Met Gln Ala Ser Ile Glu Lys Gly Gly Ser Leu
                250                 255                 260

ccc aaa gtg gaa gcc aaa ttc atc aat tat gtg aag aat tgc ttc cgg        931
Pro Lys Val Glu Ala Lys Phe Ile Asn Tyr Val Lys Asn Cys Phe Arg
            265                 270                 275

atg act gac caa gag gct att caa gat ctc tgg cag tgg agg aag tct     979
Met Thr Asp Gln Glu Ala Ile Gln Asp Leu Trp Gln Trp Arg Lys Ser
        280                 285                 290

ctt taa gaaaatagtt taaacaattt gttaaaaaat tttccgtctt atttcatttc     1035
Leu

tgtaacagtt gatatctggc tgtccttttt ataatgcaga gtgagaactt tccctaccgt     1095

gtttgataaa tgttgtccag gttctattgc caagaatgtg ttgtccaaaa tgcctgttta     1155

gtttttaaag atggaactcc accctttgct tggttttaag tatgtatgga atgttatgat     1215

aggacatagt agtagcggtg gtcagacatg gaaatggtgg ggagacaaaa atatacatgt     1275

gaaataaaac tcagtatttt aataaaataa aaaaaaaaaa aaaaaaaaaa aaaaaaaa     1333

<210> 2
<211> 294
<212> PRT
<213> Homo sapiens

<400> 2

Met Glu Asp Ser Met Asp Met Asp Met Ser Pro Leu Arg Pro Gln Asn
1               5                   10                  15

Tyr Leu Phe Gly Cys Glu Leu Lys Ala Asp Lys Asp Tyr His Phe Lys
            20                  25                  30

Val Asp Asn Asp Glu Asn Glu His Gln Leu Ser Leu Arg Thr Val Ser
        35                  40                  45

Leu Gly Ala Gly Ala Lys Asp Glu Leu His Ile Val Glu Ala Glu Ala
    50                  55                  60

Met Asn Tyr Glu Gly Ser Pro Ile Lys Val Thr Leu Ala Thr Leu Lys
65                  70                  75                  80

Met Ser Val Gln Pro Thr Val Ser Leu Gly Gly Phe Glu Ile Thr Pro
                85                  90                  95

Pro Val Val Leu Arg Leu Lys Cys Gly Ser Gly Pro Val His Ile Ser

            100                 105                 110

Gly Gln His Leu Val Ala Val Glu Glu Asp Ala Glu Ser Glu Asp Glu
        115                 120                 125

Glu Glu Glu Asp Val Lys Leu Leu Ser Ile Ser Gly Lys Arg Ser Ala
    130                 135                 140

Pro Gly Gly Gly Ser Lys Val Pro Gln Lys Lys Val Lys Leu Ala Ala
145                 150                 155                 160

Asp Glu Asp Asp Asp Asp Asp Asp Glu Glu Asp Asp Asp Glu Asp Asp
                165                 170                 175

Asp Asp Asp Asp Phe Asp Asp Glu Glu Ala Glu Glu Lys Ala Pro Val
            180                 185                 190

Lys Lys Ser Ile Arg Asp Thr Pro Ala Lys Asn Ala Gln Lys Ser Asn
        195                 200                 205

Gln Asn Gly Lys Asp Ser Lys Pro Ser Ser Thr Pro Arg Ser Lys Gly
    210                 215                 220

Gln Glu Ser Phe Lys Lys Gln Glu Lys Thr Pro Lys Thr Pro Lys Gly
225                 230                 235                 240

Pro Ser Ser Val Glu Asp Ile Lys Ala Lys Met Gln Ala Ser Ile Glu
                245                 250                 255

Lys Gly Gly Ser Leu Pro Lys Val Glu Ala Lys Phe Ile Asn Tyr Val
            260                 265                 270

Lys Asn Cys Phe Arg Met Thr Asp Gln Glu Ala Ile Gln Asp Leu Trp
        275                 280                 285

Gln Trp Arg Lys Ser Leu
    290

<210> 3
<211> 12
<212> PRT
<213> Artificial

<220>
<223> primer

<400> 3

Cys Val Met Ser Phe Glu Leu Leu Pro Leu Asp Ser
1               5                   10


<210> 4
<211> 8
<212> PRT
<213> Homo sapiens

<400> 4

Ala Asp Lys Asp Tyr His Phe Lys
1               5


<210> 5
<211> 13
<212> PRT
<213> Homo sapiens

<400> 5

Val Asp Asn Asp Glu Asn Glu His Gln Leu Ser Leu Arg
1               5                   10


<210> 6
<211> 9
<212> PRT
<213> Homo sapiens

<400> 6

Thr Val Ser Leu Gly Ala Gly Ala Lys
1               5


<210> 7
<211> 7
<212> PRT
<213> Homo sapiens

<400> 7

Val Thr Leu Ala Thr Leu Lys
1               5


<210> 8
<211> 13
<212> PRT
<213> Homo sapiens

<400> 8

Ser Ala Pro Gly Gly Gly Ser Lys Val Pro Gln Lys Lys
1               5                   10


<210> 9
<211> 4
<212> PRT
<213> Homo sapiens

<400> 9

Val Pro Gln Lys
1


<210> 10
<211> 15
<212> PRT
<213> Homo sapiens

<400> 10

Asp Thr Pro Ala Lys Asn Ala Gln Lys Ser Asn Gln Asn Gly Lys
1               5                   10                  15


<210> 11
<211> 13
<212> PRT
<213> Homo sapiens

<400> 11

Asn Ala Gln Lys Ser Asn Gln Asn Gly Lys Asp Ser Lys
1               5                   10

<210>  12
<211>  11
<212>  PRT
<213>  Homo sapiens

<400>  12

Asp Ser Lys Pro Ser Ser Thr Pro Arg Ser Lys
1               5                   10


<210>  13
<211>  14
<212>  PRT
<213>  Homo sapiens

<400>  13

Pro Ser Ser Thr Pro Arg Ser Lys Gly Gln Glu Ser Phe Lys
1               5                   10


<210>  14
<211>  6
<212>  PRT
<213>  Homo sapiens

<400>  14

Gly Gln Glu Ser Phe Lys
1               5


<210>  15
<211>  4
<212>  PRT
<213>  Homo sapiens

<400>  15

Lys Gln Glu Lys
1


<210>  16
<211>  9
<212>  PRT
<213>  Homo sapiens

<400>  16

Gly Pro Ser Ser Val Glu Asp Ile Lys
1               5

<210>  17
<211>  13
<212>  PRT
<213>  Homo sapiens

<400>  17

Met Gln Ala Ser Ile Glu Lys Gly Gly Ser Leu Pro Lys
1               5                   10

<210>  18
<211>  10
<212>  PRT
<213>  Homo sapiens

<400>  18

Val Glu Ala Lys Phe Ile Asn Tyr Val Lys
1               5                   10

<210>  19
<211>  10
<212>  PRT
<213>  Homo sapiens

<400>  19

Phe Ile Asn Tyr Val Lys Asn Cys Phe Arg
1               5                   10

<210>  20
<211>  14
<212>  PRT
<213>  Homo sapiens

<400>  20

Met Thr Asp Gln Glu Ala Ile Gln Asp Leu Trp Gln Trp Arg
1               5                   10

<210>  21
<211>  7
<212>  PRT

<213>  Homo sapiens

<400>  21

Val Thr Leu Ala Thr Leu Lys
1               5



<210>  22
<211>  7
<212>  PRT
<213>  Homo sapiens

<400>  22

Leu Leu Ser Ile Ser Gly Lys
1               5



<210>  23
<211>  11
<212>  PRT
<213>  Homo sapiens

<400>  23

Gly Pro Ser Ser Val Glu Asp Ile Lys Ala Lys
1               5                   10



<210>  24
<211>  6
<212>  PRT
<213>  Homo sapiens

<400>  24

Phe Ile Asn Tyr Val Lys
1               5

SEQUENCE LISTING

<110> LOCOMOGENE,INC.
ST. MARIANNA UNIVERSITY SCHOOL OF MEDICINE

<120> CARCINOSTATIC METHOD USING BRCA1-BARD1 PATHWAY

<130> N.98825 GCW

<140> EP05709922.8
<141> 2005-02-02

<150> PCT/JP05/01870
<151> 2005-02-02

<150> US60/541287
<151> 2004-02-02

<160> 24

<170> PatentIn version 3.2

<210> 1
<211> 1333
<212> DNA
<213> Homo sapiens

<220>
<221> CDS
<222> (101)..(985)

<400> 1
ggggccctgg tgtgattccg tcctgcgcgg ttgttctctg gagcagcgtt cttttatctc 60

cgtccgcctt ctctcctacc taagtgcgtg ccgccacccg atg gaa gat tcg atg 115
Met Glu Asp Ser Met
1               5

gac atg gac atg agc ccc ctg agg ccc cag aac tat ctt ttc ggt tgt 163
Asp Met Asp Met Ser Pro Leu Arg Pro Gln Asn Tyr Leu Phe Gly Cys
                10                  15                  20

gaa cta aag gcc gac aaa gat tat cac ttt aag gtg gat aat gat gaa 211
Glu Leu Lys Ala Asp Lys Asp Tyr His Phe Lys Val Asp Asn Asp Glu
            25                  30                  35

aat gag cac cag tta tct tta aga acg gtc agt tta ggg gct ggt gca 259
Asn Glu His Gln Leu Ser Leu Arg Thr Val Ser Leu Gly Ala Gly Ala
        40                  45                  50

aag gat gag ttg cac att gtt gaa gca gag gca atg aat tac gaa ggc 307
Lys Asp Glu Leu His Ile Val Glu Ala Glu Ala Met Asn Tyr Glu Gly
    55                  60                  65

agt cca att aaa gta aca ctg gca act ttg aaa atg tct gta cag cca 355
Ser Pro Ile Lys Val Thr Leu Ala Thr Leu Lys Met Ser Val Gln Pro
70                  75                  80                  85

acg gtt tcc ctt ggg ggc ttt gaa ata aca cca cca gtg gtc tta agg     403
Thr Val Ser Leu Gly Gly Phe Glu Ile Thr Pro Pro Val Val Leu Arg
            90                  95                  100

ttg aag tgt ggt tca ggg cca gtg cat att agt gga cag cac tta gta     451
Leu Lys Cys Gly Ser Gly Pro Val His Ile Ser Gly Gln His Leu Val
        105                 110                 115

gct gtg gag gaa gat gca gag tca gaa gat gaa gag gag gag gat gtg     499
Ala Val Glu Glu Asp Ala Glu Ser Glu Asp Glu Glu Glu Glu Asp Val
    120                 125                 130

aaa ctc tta agt ata tct gga aag cgg tct gcc cct gga ggt ggt agc     547
Lys Leu Leu Ser Ile Ser Gly Lys Arg Ser Ala Pro Gly Gly Gly Ser
135                 140                 145

aag gtt cca cag aaa aaa gta aaa ctt gct gct gat gaa gat gat gac     595
Lys Val Pro Gln Lys Lys Val Lys Leu Ala Ala Asp Glu Asp Asp Asp
150                 155                 160                 165

gat gat gat gaa gag gat gat gat gaa gat gat gat gat gat gat ttt     643
Asp Asp Asp Glu Glu Asp Asp Asp Glu Asp Asp Asp Asp Asp Asp Phe
            170                 175                 180

gat gat gag gaa gct gaa gaa aaa gcg cca gtg aag aaa tct ata cga     691
Asp Asp Glu Glu Ala Glu Glu Lys Ala Pro Val Lys Lys Ser Ile Arg
        185                 190                 195

gat act cca gcc aaa aat gca caa aag tca aat cag aat gga aaa gac     739
Asp Thr Pro Ala Lys Asn Ala Gln Lys Ser Asn Gln Asn Gly Lys Asp
    200                 205                 210

tca aaa cca tca tca aca cca aga tca aaa gga caa gaa tcc ttc aag     787
Ser Lys Pro Ser Ser Thr Pro Arg Ser Lys Gly Gln Glu Ser Phe Lys
215                 220                 225

aaa cag gaa aaa act cct aaa aca cca aaa gga cct agt tct gta gaa     835
Lys Gln Glu Lys Thr Pro Lys Thr Pro Lys Gly Pro Ser Ser Val Glu
230                 235                 240                 245

gac att aaa gca aaa atg caa gca agt ata gaa aaa ggt ggt tct ctt     883
Asp Ile Lys Ala Lys Met Gln Ala Ser Ile Glu Lys Gly Gly Ser Leu
            250                 255                 260

ccc aaa gtg gaa gcc aaa ttc atc aat tat gtg aag aat tgc ttc cgg     931
Pro Lys Val Glu Ala Lys Phe Ile Asn Tyr Val Lys Asn Cys Phe Arg
        265                 270                 275

atg act gac caa gag gct att caa gat ctc tgg cag tgg agg aag tct     979
Met Thr Asp Gln Glu Ala Ile Gln Asp Leu Trp Gln Trp Arg Lys Ser
    280                 285                 290

ctt taa gaaaatagtt taaacaattt gttaaaaaat tttccgtctt atttcatttc     1035
Leu

tgtaacagtt gatatctggc tgtccttttt ataatgcaga gtgagaactt tccctaccgt     1095

gtttgataaa tgttgtccag gttctattgc caagaatgtg ttgtccaaaa tgcctgttta 1155

gtttttaaag atggaactcc accctttgct tggttttaag tatgtatgga atgttatgat 1215

aggacatagt agtagcggtg gtcagacatg gaaatggtgg ggagacaaaa atatacatgt 1275

gaaataaaac tcagtatttt aataaaataa aaaaaaaaaa aaaaaaaaaa aaaaaaa 1333

<210> 2
<211> 294
<212> PRT
<213> Homo sapiens

<400> 2

Met Glu Asp Ser Met Asp Met Asp Met Ser Pro Leu Arg Pro Gln Asn
1               5                   10                  15

Tyr Leu Phe Gly Cys Glu Leu Lys Ala Asp Lys Asp Tyr His Phe Lys
            20                  25                  30

Val Asp Asn Asp Glu Asn Glu His Gln Leu Ser Leu Arg Thr Val Ser
        35                  40                  45

Leu Gly Ala Gly Ala Lys Asp Glu Leu His Ile Val Glu Ala Glu Ala
    50                  55                  60

Met Asn Tyr Glu Gly Ser Pro Ile Lys Val Thr Leu Ala Thr Leu Lys
65                  70                  75                  80

Met Ser Val Gln Pro Thr Val Ser Leu Gly Gly Phe Glu Ile Thr Pro
                85                  90                  95

Pro Val Val Leu Arg Leu Lys Cys Gly Ser Gly Pro Val His Ile Ser
            100                 105                 110

Gly Gln His Leu Val Ala Val Glu Glu Asp Ala Glu Ser Glu Asp Glu
        115                 120                 125

Glu Glu Glu Asp Val Lys Leu Leu Ser Ile Ser Gly Lys Arg Ser Ala
    130                 135                 140

Pro Gly Gly Gly Ser Lys Val Pro Gln Lys Lys Val Lys Leu Ala Ala
145                 150                 155                 160

Asp Glu Asp Asp Asp Asp Asp Asp Glu Glu Asp Asp Asp Glu Asp Asp

                165                 170                 175

Asp Asp Asp Asp Phe Asp Asp Glu Glu Ala Glu Glu Lys Ala Pro Val
            180                 185                 190

Lys Lys Ser Ile Arg Asp Thr Pro Ala Lys Asn Ala Gln Lys Ser Asn
        195                 200                 205

Gln Asn Gly Lys Asp Ser Lys Pro Ser Ser Thr Pro Arg Ser Lys Gly
    210                 215                 220

Gln Glu Ser Phe Lys Lys Gln Glu Lys Thr Pro Lys Thr Pro Lys Gly
225                 230                 235                 240

Pro Ser Ser Val Glu Asp Ile Lys Ala Lys Met Gln Ala Ser Ile Glu
                245                 250                 255

Lys Gly Gly Ser Leu Pro Lys Val Glu Ala Lys Phe Ile Asn Tyr Val
            260                 265                 270

Lys Asn Cys Phe Arg Met Thr Asp Gln Glu Ala Ile Gln Asp Leu Trp
        275                 280                 285

Gln Trp Arg Lys Ser Leu
    290

<210> 3
<211> 12
<212> PRT
<213> Artificial

<220>
<223> primer

<400> 3

Cys Val Met Ser Phe Glu Leu Leu Pro Leu Asp Ser
1               5                   10

<210> 4
<211> 8
<212> PRT
<213> Homo sapiens

<400> 4

Ala Asp Lys Asp Tyr His Phe Lys
1               5

<210> 5
<211> 13
<212> PRT
<213> Homo sapiens

<400> 5

Val Asp Asn Asp Glu Asn Glu His Gln Leu Ser Leu Arg
1               5                   10


<210> 6
<211> 9
<212> PRT
<213> Homo sapiens

<400> 6

Thr Val Ser Leu Gly Ala Gly Ala Lys
1               5


<210> 7
<211> 7
<212> PRT
<213> Homo sapiens

<400> 7

Val Thr Leu Ala Thr Leu Lys
1               5


<210> 8
<211> 13
<212> PRT
<213> Homo sapiens

<400> 8

Ser Ala Pro Gly Gly Gly Ser Lys Val Pro Gln Lys Lys
1               5                   10


<210> 9
<211> 4
<212> PRT
<213> Homo sapiens

<400> 9

Val Pro Gln Lys
1


<210> 10

<211> 15
<212> PRT
<213> Homo sapiens

<400> 10

Asp Thr Pro Ala Lys Asn Ala Gln Lys Ser Asn Gln Asn Gly Lys
1               5                   10                  15


<210> 11
<211> 13
<212> PRT
<213> Homo sapiens

<400> 11

Asn Ala Gln Lys Ser Asn Gln Asn Gly Lys Asp Ser Lys
1               5                   10


<210> 12
<211> 11
<212> PRT
<213> Homo sapiens

<400> 12

Asp Ser Lys Pro Ser Ser Thr Pro Arg Ser Lys
1               5                   10


<210> 13
<211> 14
<212> PRT
<213> Homo sapiens

<400> 13

Pro Ser Ser Thr Pro Arg Ser Lys Gly Gln Glu Ser Phe Lys
1               5                   10


<210> 14
<211> 6
<212> PRT
<213> Homo sapiens

<400> 14

Gly Gln Glu Ser Phe Lys
1               5


<210> 15
<211> 4
<212> PRT
<213> Homo sapiens

<400> 15

Lys Gln Glu Lys
1


<210> 16
<211> 9
<212> PRT
<213> Homo sapiens

<400> 16

Gly Pro Ser Ser Val Glu Asp Ile Lys
1               5


<210> 17
<211> 13
<212> PRT
<213> Homo sapiens

<400> 17

Met Gln Ala Ser Ile Glu Lys Gly Gly Ser Leu Pro Lys
1               5                   10


<210> 18
<211> 10
<212> PRT
<213> Homo sapiens

<400> 18

Val Glu Ala Lys Phe Ile Asn Tyr Val Lys
1               5                   10


<210> 19
<211> 10
<212> PRT
<213> Homo sapiens

<400> 19

Phe Ile Asn Tyr Val Lys Asn Cys Phe Arg
1               5                   10


<210> 20
<211> 14
<212> PRT
<213> Homo sapiens

<400> 20

Met Thr Asp Gln Glu Ala Ile Gln Asp Leu Trp Gln Trp Arg
1               5                   10

<210> 21
<211> 7
<212> PRT
<213> Homo sapiens

<400> 21

Val Thr Leu Ala Thr Leu Lys
1               5

<210> 22
<211> 7
<212> PRT
<213> Homo sapiens

<400> 22

Leu Leu Ser Ile Ser Gly Lys
1               5

<210> 23
<211> 11
<212> PRT
<213> Homo sapiens

<400> 23

Gly Pro Ser Ser Val Glu Asp Ile Lys Ala Lys
1               5                   10

<210> 24
<211> 6
<212> PRT
<213> Homo sapiens

<400> 24

Phe Ile Asn Tyr Val Lys
1               5

## Description

### TECHNICAL FIELD

This invention relates to a method of polyubiquitinating a nucleophsmin including the reaction of nucleophosmin with BRCA1-BARD1.

### BACKGROUND ART

*BRCA1*, which is a tumor suppressor gene for breast and ovarian cancers, is one of the most important genes in the field of breast cancer research¹⁻⁴. Recently, the present inventors have found that BRCA1 and BARD1 form a RING heterodimeric ubiquitin ligase and that the activity of this ligase is completely inactivated by a missense mutation in *BRCA1* which causes familial breast cancer^{1,9}. Further, the inventors have reported that polyubiquitin chains catalyzed by BRCA1-BARD1 are not the conventional Lys⁴⁸-linked ubiquitin chains which function as a signal for proteolysis by 26S proteasome, but are Lys⁶-linked ubiquitin chains that have not yet been reported in the past and that these polyubiquitin chains are deubiquitinated *in vitro* by 26S proteasome^{2,3}. However, it has not been elucidated how the activity of the ubiquitin ligase is involved in the tumor suppressing function of BRCA1. The major reasons are that the substrate for the ligase activity has not been identified and that the upstream signals that control the activity have not been clearly understood.

It has been reported that nucleophosmin (NPM) is present in the centrosome during the G1 phase and that centrosome replication begins when NPM has been phosphorylated by CDK2-cyclin E during the G1/S transition phase and separated from the centrosome^{7, 8}. Since NPM returns to the centrosome at the time of mitosis and the centrosome of the daughter cells after mitosis has NPM, it has been indicated that NPM may be a licensing factor for centrosome replication. However, it has not been elucidated how NPM returns to the centrosome during the mitotic phase. On the other hand, it is known that deletion of BRCA1 causes excessive replication of the centrosome, which makes the genome unstable. This mechanism has not been clearly understood yet.

### DISCLOSURE OF THE INVENTION

As mentioned above, it is not clear how the BRCA1-BARD1 ligase regulates various cellular processes such as DNA repair, cell-cycle progression, and centrosome replication⁴⁻⁶.

The purpose of the present invention is to carry out polyubiquitination of nucleophosmin including the reaction of nucleophosmin with BRCA1-BARD1. The present inventors thoroughly investigated in an attempt to solve the aforementioned subject of concern and found that BRCA1-BARD1 bind to and catalyze the polyubiquitination of nuclear phosphoprotein, nucleophosmin/B23 (NPM), which causes NPM stabilization.

In one aspect of the invention, a method of polyubiquitinating a nucleophosmin comprised by reacting the nucleophosmin with BRCA1-BARD1 is provided. According to this method, the polyubiquitination may be carried out *in vitro* or *in vivo.*

In one aspect of the invention, a method of stabilizing nucleophosmin comprising the polyubiquitination of nucleophosmin is provided.

In another aspect of the invention, a method of inhibiting polyubiquitination of nucleophosmin comprising phosphorylating a BARD1 using CDK2-cyclin E and/or CDK2-cyclin A, and a method of dissociating BRCA1-BARD1, and a method of inactivating the activity of ubiquitin ligase are provided. In the present methods, phosphorylation sites of the BARD1 are at least 3 sites selected from the group consisting of S 148, S251, S288 and T299, and the said sites can be S 148, S288 and T299 and can be S148, S251, S288 and T299.

The other aspect of the invention is a method of transporting BRCA1 from nucleus to cytoplasm expression comprising of both BRCA1 and CDK2-cyclin E and/or CDK1-cyclin A.

### BRIEF DESCRIPTION OF THE DRAWINGS

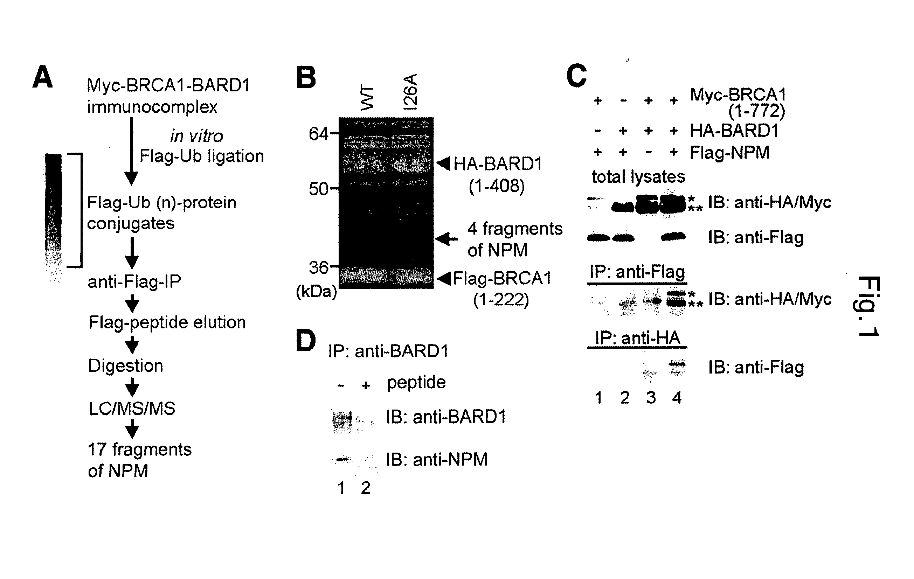

Fig. 1 shows that NPM was identified as a substrate of the BRCA1-BARD1 ligase by two different screenings. [*] and [**] represent Myc-BRCA1 (1-772) and HA-BARD1, respectively.

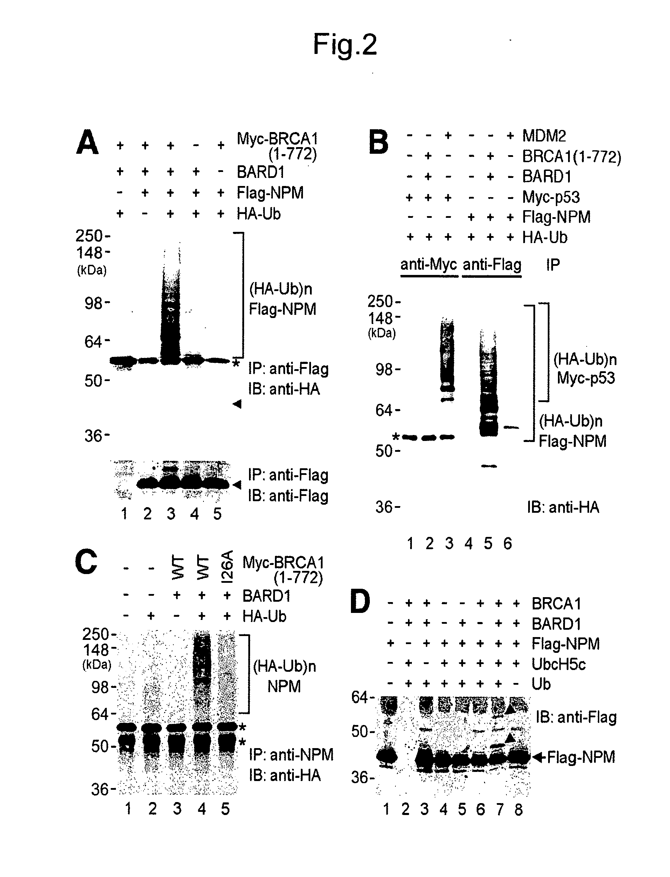

Fig. 2 is a diagram showing NPM ubiquitination by BRCA1-BARD1. The arrowheads in A indicate the positions of non-ubiquitinated Flag-NPM. The arrowheads in D indicate the positions of ubiquitinated Flag-NPM. [*] represents IgG.

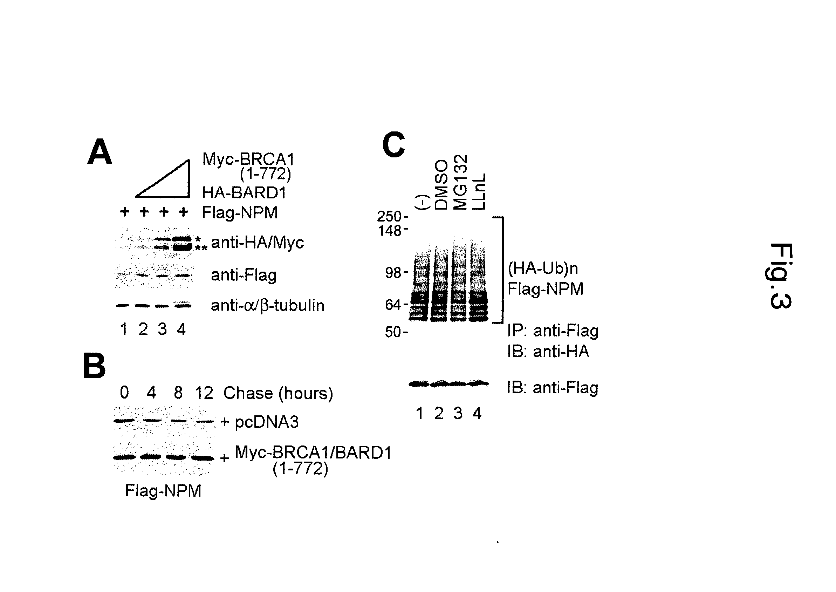

Fig. 3 is a diagram showing that NPM ubiquitination by BRCA1-BARD1 is not a signal for the proteolysis by proteasome. In A, 293T cells were transfected with indicated plasmids: 0.5 µg of Flag-NPM in Lanes 1-4, 0.05 µg of Myc-BRCA1¹⁻⁷⁷² and HA-BARD1 in Lane 2, 0.25 µg of Myc-BRCA1¹⁻⁷⁷² and HA-BARD1 in Lane 3, and 1 µg of Myc-BRCA1¹⁻⁷⁷² and HA-BARD1 in Lane 4.

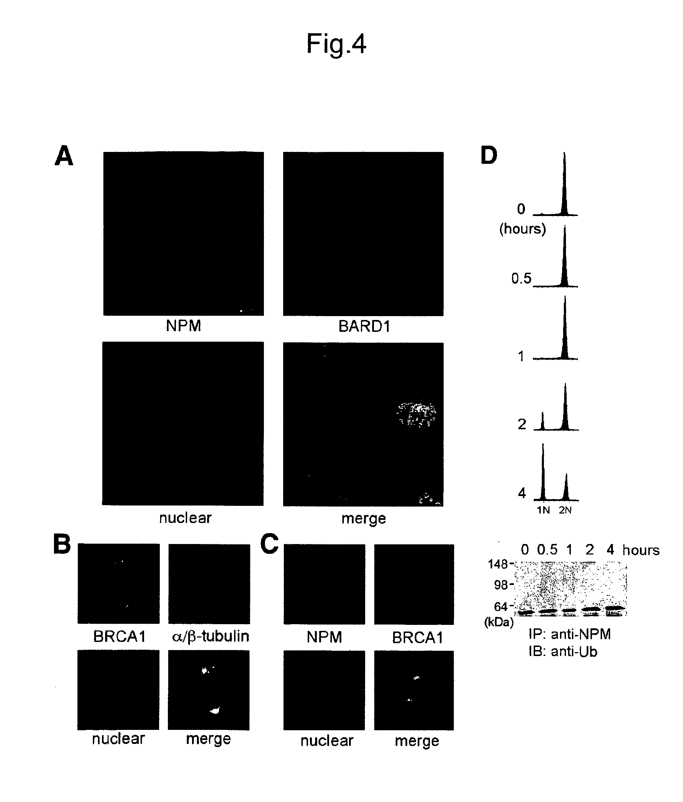

Fig. 4 is a diagram showing the colocalization of NPM and BRCA1-BARD1 and NPM ubiquitination during mitosis. The merge in A shows images of two proteins (NPM and BARD1) are overlapped. The merge in B and C shows images of two proteins (B: BRCA1 and α/β-tubulin C: NPM and BRCA1) and the nucleus are overlapped. D shows the DNA contents monitored by flow cytometry in the upper portion.

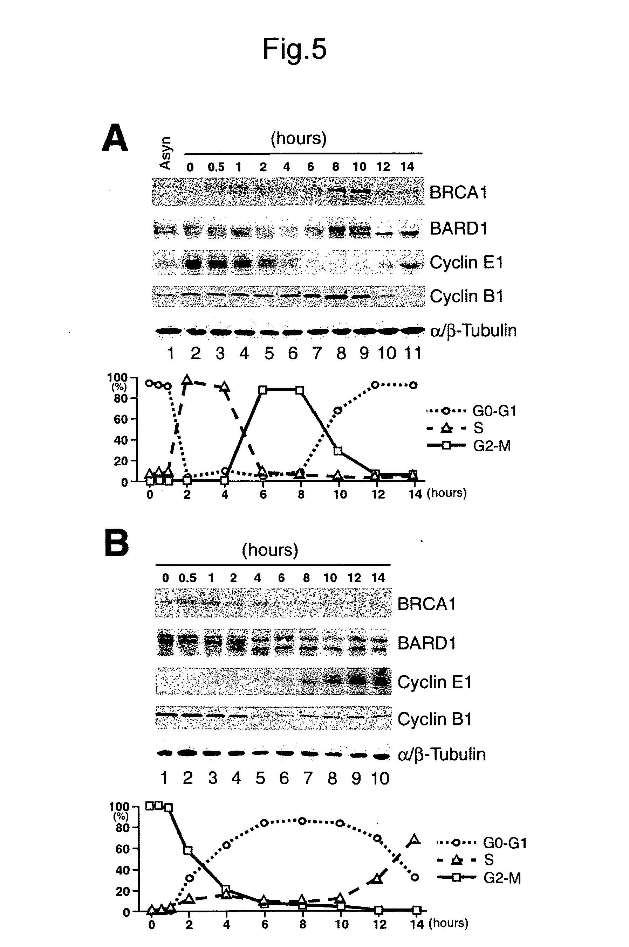

Fig. 5 is a diagram showing the expression depending upon the cell-cycle of BARD1. A shows cell cycle synchronization of the double thymidine block and B shows cell cycle synchronization of the thymidine nocodazole block. The upper portion of each graph shows the results of the immune blot over time for the expression of each protein after releasing blocks and the lower portion of the graph shows the results monitored by FACS analysis for the courses of the cell cycle after releasing the inhibition. Asyn indicates asynchronous cells.

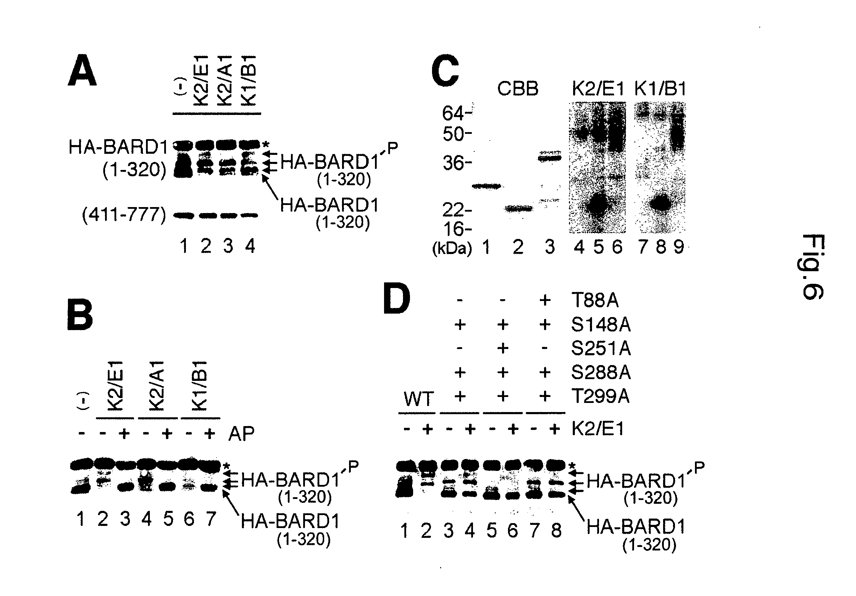

Fig. 6 is a diagram showing that CDK2-cyclin A1/E1 and CDK1-cyclin B1 phosphorylates the NH₂-terminal side. * represents IgG, WT represents a wild type, K2/E1 represents CDK2-cyclin E1, K2/A1 represents CDK2-cyclin A1, K1/B1 represents CDK1-cyclin B1, and that HA-BARD1^{-P} represents phosphorylated HA-BARD1.

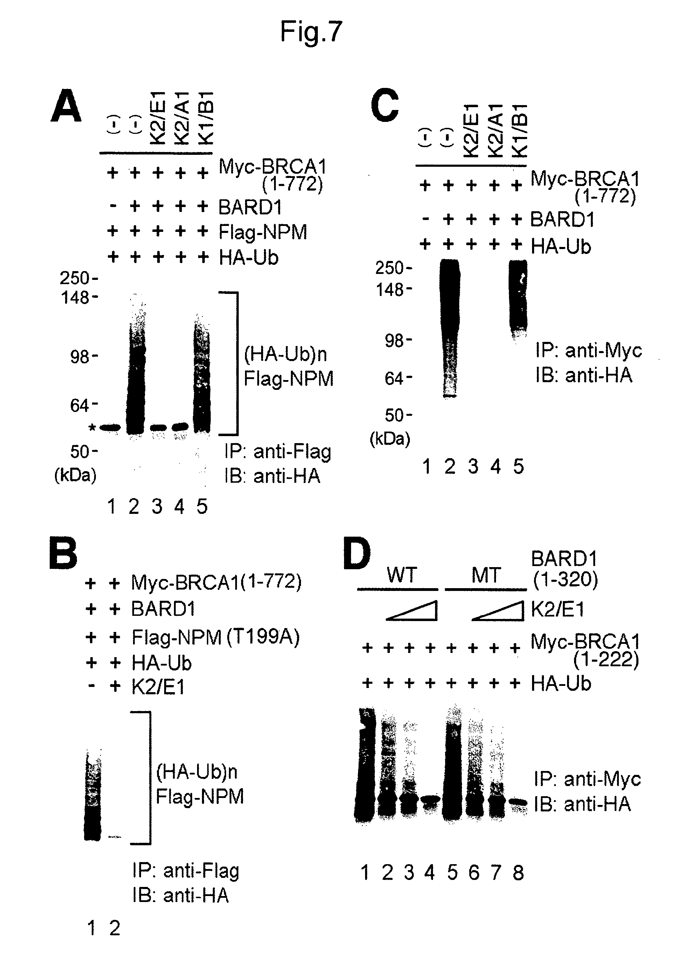

Fig. 7A-C are graphs showing that CDK-cyclin E and CDK-cyclin A inhibit NPM ubiquitination. K2/E represents CDK2-cyclin E, K2/A represents CDK2-cyclin A, and K1/B represents CDK1-cyclin B. D is a graph showing that CDK2-cyclin E1 and CDK2-cyclin A1 inhibit the ubiquitin ligase activity of BRCA1-BARD1 with concentration dependence. CDK2-cyclin E1 content of 0.2 µg was added in the lanes 2 and 6, CDK2-cyclin E1 content of 0.6 µg was added in the lanes 3 and 7, and CDK2-cyclin E1 content of 2 µg was added in the lanes 4 and 8. WT represents a wild type and MT represents BARD1^{S148A/S251A/S288A/T299A}.

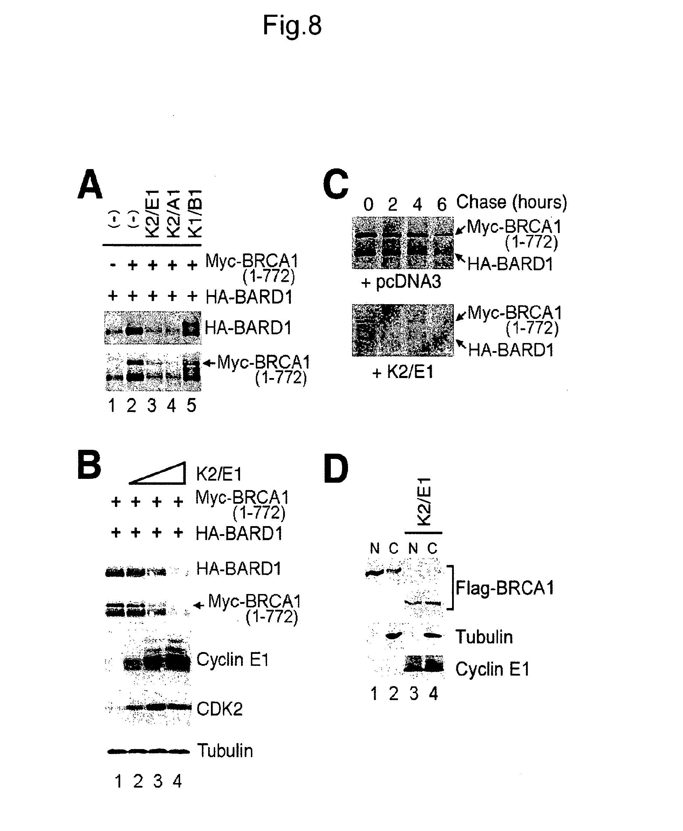

Fig. 8 is a graph showing that BRCA1 and BARD1 are transported to cytoplasm and are unstablized due to the coexpression of CDK2-cyclin E1. A: anti-HA antibodies (upper stage) and anti-Myc antibodies after the anti-HA antibody reaction (lower stage) were analyzed by re-probed immunoblotting. B: CDK2-cyclin E1 was added in 0.1 µg in lane 2, 0.5 µg in lane 3, and 1.5 µg in lane 4. Myc-BRCA¹⁻⁷⁷² and HA-BARD1 were added in 1 µg in every lane. The steady state of each protein was analyzed by a immunoblotting using re-probe for anti-HA antibodies in the upper stage and anti-Myc after the anti-HA antibody reaction. C: Myc-BRCA1¹⁻⁷⁷², HA-BARD1 and pcDNA3 vector in the upper stage and Myc- BRCA1¹⁻⁷⁷², HA-BARD1 and CDK2-cyclin E1 in the lower stage were forced expressed in the 293T cells. D: FLAG-BRCA1, HA-BARD1 and pcDNA3 vector were used in lanes 1 and 2, and FLAG-BRCA1, HA-BARD1 and CDK2-cyclin E1 were used in lanes 3 and 4. Also, N represents the nucleus and C represents cytoplasm. The second stage shows the case of longer exposure than the upper stage.

### BEST MODE FOR CARRYING OUT THE INVENTION

The present invention relates to a method of polyubiquitinating a nucleophosmin comprising of reacting nucleophosmin with BRCA1-BARD1. NPM coexists with BRCA1 at the spindle pole at the M phase where it is ubiquitinated. It has been known that CDK2-cyclin E and CDK2-cyclin A phosphorylate NPM during the G1/S transition phase and S phase and NPM is dissociated from the centrasome^{7,8}. To our interest, CDK2 also phosphorylates BARD1. As a result, dissociation of BRCA1-BARD1 occurs, thus, NPM ubiquitination by BRCA1-BARD1 is suppressed. Due to deletion of BRCA1, excessive replications of the centrosome and chromosome distribution abnormalities occur. This new CDK2-BRCA1-NPM pathway was suggested to be the mechanism of playing the role of BRCA1 in the replication of the centrosome. This invention will be described in detail below.

### 1. Nucleophosmin, BRCA1 and BARD1

Nucleophosmin in the present invention (hereinafter referred to as "NPM") is a substrate of BRCA1-BARD1 ubiquitin ligase. It has a variety of biological activities in all cells such as ribosome biosynthesis, apoptosis inhibition, and histone chaperone functions, etc. For example, it is included nucleophosmin/B23/NO38 (NPM). The inventors have identified nucleophosmin/B23/NO 38 (NPM) as the substrate for BRCA1-BARD1 ubiquitin ligase by two screening methods using a mass spectrometer (LC/MS/MS). NPM coexists with BRCA1 at the spindle pole during the mitotic phase (M phase) and is ubiquitinated. It has been known that CDK2-cyclin E and CDK2-cyclin A phosphorylate NPM and dissociate NPM from the centrosome, which is result in centrosome is divided into the daughter centrosomes^{7,8} during the G1/S transition phase and S phase. During the S phase, NPM cannot bind with the centrosome. Meanwhile, the daughter centrosomes grow to the centrosome and subsequently to the two spindle poles. As a control mechanism of centrosome replication, a CDK2-BRCA1-NPM pathway exists and NPM ubiquitination by BRCA1-BARD1 plays an important role in the re-localization of NPM to the spindle poles during the mitotic phase.

BRCA1 is a tumor suppressor gene for breast and ovarian cancers and is one of the most important genes in the field of breast cancer research. BRCA1 and BARD1 form a RING heterodimer to acquire high ubiquitin ligase activity¹⁻⁴. Targeted disruption of the BRCA1 gene in mice resulted in centrosome hyperamplification and genomic instability^{13,14}.

During mitosis, BRCA1 is localized to the centrosome through its binding to gamma-tubulin^{15, 16}.

BARD1 was identified as a RING finger protein bound to BRCA1 (BRCA1 associated Ring Domain 1).

BRCA1-BARD1 form a RING heterodimeric ubiquitin ligase and the activity of this ligase is completely inactivated due to missense mutation in BRCA1, which causes familial breast cancer^{1,9}

BRCA1-BARD1 ligase regulates a variety of cellular processes such as DNA repair, cell-cycle progression and centrosome replication. According to the immunocytostaining experiments, the behaviors of NPM, BRCA1 and BARD1 at various phases of cellular cycle were found to be as follows. Namely, NPM was present in the nucleosome during the interphase, whereas BRCA1 and BARD1 were present in the nucleus other than the nucleosome. However, they coexisted around the nucleus near the spindle body and in the centrosome (spindle pole) during the mitotic phase. According to the tests using Hela cells synchronized by a thymidine-nocodazole block at the time of mitotic phase of the cell cycle, NPM was found to be polyubiquitinated in a short time during the transition phase from the mitotic phase to the G1 phase. In order to observe subcellular localization and its coexistence with NPM during the cell cycle, a rabbit polyclonal antibody specific to the C-terminal of BARD1 can be utilized by preparing it. Synchronization of the cellular cycle is monitored by flowcytometry and *in vivo* NPM ubiquitination was assessed by IP-Western analysis.

Polyubiquitin chains catalyzed by BRCA1-BARD1 are not the conventional Lys⁴⁸⁻linked ubiquitin chains which function as a signal for proteolysis by 26S proteasome, but Lys⁶-linked ubiquitin chains that have not yet been reported in the past and that these polyubiquitin chains are de-ubiquitinated *in vitro* by 26S proteasome^{2,3}. NPM is ubiquitinated and stabilized by BRCA1-BARD1 *in vivo,* but this ubiquitination does not function as a signal for the 26S proteasome-dependent proteolysis.

It is noteworthy¹⁷ that NPM redistributes to the spindle poles during the mitotic phase. NPM ubiquitination by BRCA1-BARD1 is important for redistribution so that its absence may cause excessive replication of the centrosome. Furthermore, many functions attributed to NPM such as upregulation after DNA damage¹⁸, a role during the cell cycle and apoptosis^{19,20}, and implications in chromatin remodeling^{21,22} overlap with the functions of BRCA1. Therefore, NPM ubiquitination by BRCA1-BARD1 can be the mechanism carried by BRCA1 as one of the known functions.

### 2. Ubiquitination/polyubiquitination

Ubiquitination is a process wherein enzymes such as ubiquitin activation enzyme (E1), ubiquiting binding enzyme (E2), and ubiquitin ligase (E3), are coordinated to bind ubiquitin sequentially onto the protein as a substrate. The physiological implication of ubiquitination is to lead the substrate to the 26S proteasome-dependent proteolysis, which is due to ubiquitin-ubiquitin links via Lys-48 on ubiquitin^{11, 12}. However, the present inventors and other researchers discovered recently that BRCA1-BARD1 catalyzes the formation of a non-conventional Lys-6 linked type polyubiquitin chain. It is deubiquitinated by purified 26S proteasome *in vitro* instead of being targeted for proteolysis^{2,3}.

"Self ubiquitination" used in this specification means that due to the presence of ubiquitin ligase activity of BRCA1-BARD1, BRCA1 itself becomes a substrate protein for ubiquitination so that BRCA 1 itself binds ubiquitin rather than using other ubiquitin ligase.

NPM ubiquitination does not function as a signal for the 26S proteasome-dependent proteolysis. Whether or not BRCA1-BARD1-mediated NPM ubiquitination targets NPM for proteolysis can be determined by measuring its stability *in vivo.* In the case of expression with NPM alone and in the case of coexpression in the presence of BRCA1-BARD1, if the stability of NPM is higher in the case of coexpression, NPM ubiquitination by BRCA1-BARD1 is not a signal for the proteasome proteolysis. Whether or not BRCA1-BARD1-mediated NPM ubiquitination targets NPM for proteolysis can also be determined by the pulse-chase analysis. Also, it can be determined by treatment with proteasome inhibitors MG 132 or LLnL. In this case, if the amount of ubiquitinated Flag-NPM does not increase, BRCA1-BARD1-mediated NPM ubiquitination is not a signal for proteasome proteolysis. These finding suggest that BRCA1-BARD1-mediated NPM ubiquitination affects the function of NPM through a non-proteolytic mechanism

It is also clear that NPM is ubiquitinated *in vitro* by BRCA1-BARD1. Whether BRCA1-BARD1 ubiquitinates NPM *in vitro* can be tested in an *in vitro* system using a recombinant protein alone. For example, tagged NPM, BRCA1 and BARD 1 are incubated with E1 and E2 ubiquitins, and tagged NPM is detected by immunoblotting using an appropriate antibody. This demonstrates BRCA1-BARD1-dependent NPM ubiquitination in a purified system.

### 3. Phosphorylation of BARD 1 by CDK2-cyclin

CDK is a cyclin-dependent kinase. CDK2-cyclin E implies a complex of CDK2 with cyclin E and CDK2-cyclin A implies a complex of CDK2 with cyclin A. Both are coordinated in the cell cycle regulatory system.

A complex of CDK2-cyclin E induces a cell to pass G₁/S commitment (starting point), and a complex of CDK2-cyclin A is known to be necessary for the activation of the subsequent DNA replication apparatus. Cyclin B described later in this specification forms a CDK1 (cdc2) complex and induces the start of the miotic phase.

Both CDK2-cyclin E and CDK2-cyclin A completely abolish the ubiquitination of NPM by BRCA1-BARD1 *in vivo.* There are two possible mechanisms by which CDK2 could inhibit BRCA1-BARD1-mediated ubiquitination:
1) CDK2 phosphorylates NPM to disguise the BRCA1-BARD1 recognition elements or
2) CDK2 directly phosphorylates and inhibits the BRCA1-BARD1 ligase.

To distinguish between these two models, we first tested whether the BRCA1-BARD1 ligase is capable of ubiquitinating NPM, a mutant that abolishes the CDK2 phosphorylation sites. The aforementioned mutant is ubiquitinated by BRCA1-BARD1 ligase and the ubiquitination is inhibited by CDK2. BARD1 is a substrate of CDK2-cyclin E1 so that there is a possibility that inhibition of the BRCA1-BARD1 ubiquitin ligase activity directly affects phosphorylation of BARD1. However, BRCA1 autoubiquitination via non-phosphorylation mutant of BARD1 is also inhibited by CDK2-cyclin E1 suggesting that not all inhibition of NPM ubiquitination is attributed to phosphorylation of BARD1.

Phosphorylation of BARD1 by CDK when both BARD1 and CDK-cyclin are co-expressed can be analyzed by measuring the changes in the molecular weight of BARD1. The changes in floating positions disappear by treating the BARD1 immunoprecipitates with alkali phosphatase. This suggests that BARD1 is a direct substrate of CDK2 kinase. In this case, when using NH2 terminal segments of BARD1 and COOH terminal segments of BARD1, it is possible to determine in which areas phosphorylation of BARD1 occurs. Also, in order to investigate whether CDK directly phosphorylates BARD1, we need to investigate whether BARD 1 can become a substrate of CDK *in vitro*. It was indicated that BARD1 is phosphorylated at the NH₂ terminal side by CDK1 and CDK2 in an independent mode from the heterodimer formation of BRCA1-BARD1. Four phosphorylation sites were identified to be S¹⁴⁸/S²⁵¹/S²⁸⁸/T²⁹⁹ at the NH₂ terminal side of BARD1. The mutants at these four sites remained associated with BRCA1 when co-expressed with either CDK2-cyclin E1 or CDK1-cyclin B1, indicating little change in molecular weight. These four sites were determined to be phosphorylation sites. The aforementioned mutant of BARD1 remained to have a sensitivity for the inhibition of ubiquitin ligase activity by CDK2, and when CDK1 phosphorylated BARD1 at the same sites as CDK2, BRCA1-BARD1 ubiquitin ligase activity was not inhibited so that phosphorylation itself appeared to be not so important in the downregulation of ubiquitin ligase. The coexpression of BARD1 and CDK1-cyclin B induced phosphorylation of BARD1 *in vivo,* but no effect on binding with BARD1 and BRCA1, which suggested that the function of BRCA1-BARD1 is controlled respectively by the cell cycle kinase at different phases during the cell cycle.

Due to phosphorylation of BARD1 by the aforementioned CDK2-cyclin E and CDK2-cyclin A, BRCA1 and BARD1 were dissociated and as a result, ubiquitin ligase activity was inactivated. The effect of CDK2-cyclin E and CDK2-cyclin A on BRCA1-BARD1 heterodimer specific ligase activity can be tested by measuring BRCA1 autoubiquitination. According to this analysis, CDK1-cyclin B did not inhibit autoubiquitination of BRCA1, whereas both CDK2-cyclin E and CDK2-cyclin A completely inhibited autoubiquitination. The point to be noted is that both CDK2-cyclin E and CDK2-cyclin A decompose BRCA1-BARD1.

The expression of cyclin E at the protein level showed remarkable variations in the sporadic breast cancer and the high degree of expression levels significantly correlated to malignant prognosis²³.

According to the present invention, regarding how CDK2-cyclin E1 and A1 have an effect on the expression of protein of BRCA1 and BARD1, the steady state levels of BRCA1 and BARD1 in response to ubiquitin ligase activity decreased dramatically with CDK1-cyclin E1/A1 in a dose-dependent manner instead of with CDK1-cyclin B1. In addition, when testing the [³⁵S]-methionine-labeled cells by the pulse chase method, if a BRCA1-BARD1 complex was co-expressed by CDK2-cyclin E1, the half life in the cells decreased significantly, which suggested that a decrease in expression of protein was due to proteolysis.

According to the present invention, the presence of high levels of cyclin E indicated a decrease in the BRCA1 ubiquitin ligase activity. Therefore, BRCA1 ubiquitin ligase activity was found to be involved in sporadic breast cancer in addition to familial breast cancer.

### 4. Transport of BRCA1 from nucleus to cytoplasm

Previously, BRCA1 and BARD1 were reported previously to be degraded due to transportation from nucleus to cytoplasm. When CDK2-cyclin E1 and BRCA1 were co-expressed and the protein levels of BRCA1 in the nucleus and cytoplasm were quantitatively analyzed, it was detected that the protein level of BRCA 1 in cytoplasm was higher than the protein level in nucleus. Thus, in the presence of CDK2-cyclin E1, BRCA1 was assumed to be transported from nucleus to cytoplasm.

The present invention is explained specifically with reference to the embodiments. However, the present invention will not be limited by these embodiments for the scope of technology.

Through the present embodiments, the following specific methods were employed.

### Methods:

### Antibodies, construction of expression vector and purified proteins

Mouse monoclonal antibodies to HA (12CA5, Boehringer, Mannheim), Myc (9E10, BabCo), Flag (M2, Sigma), polyubiquitin (Affiniti), α-and β-tubulin (DMIA+BMIB, Neomarkers) and NPM (Sigma-Aldrich or Zymed) as well as rabbit polyclonal antibodies to BRCA1 (Santa Cruz c-20) were purchased commercially. The rabbit polyclonal antibody made against the C-terminal of BARD1 was generated to the synthesized peptide CVMSFELLPLDS (SEQ ID No. 3) and affinity purified before use. Its specificity for immunofluorescence in cells was confirmed by using the competing peptide.

cDNA for full length human NPM (B23.1) (SEQ ID No. 1) was amplified by PCR from a HeLa cell cDNA library using Pfx polymerase (Stratagene). The cDNA for full length human NPM (B23.1) was subcloned into the mammalian expression pcDNA3 vector in frame with the N-terminal Flag tag or into the E. coli expression pET vector in frame with the N-terminal 6x His-Flag tag. Mammalian expression plasmids for BRCA1, BARD 1 and HA-ubiquitin were prepared by the methods described in Hashizume, R. et al. The RING heterodimer BRCA1-BARD1 is ubiquitin ligase inactivated by a breast cancer-derived mutation. J. Biol. Chem. 276, 14537-14540 (2001), Nishikawa, H. et al. Mass spectrometric and mutational analyses reveal Lys-6-linked polyubiquitin chains catalyzed by BRCA1-BARD1 ubiquitin ligase. J. Biol. Chem. in press [online resource]^{1,2}

The point mutations including that for a stop codon to make truncated mutants were generated by site-directed mutagenesis (Stratagene). All plasmids used were verified by DNA sequencing. Mammalian expression plasmids for cyclins and CDKs were prepared by the methods described^{26, 27} in Ohta, T., Xiong, Y., Phosphorylation-and Skp1-independent in vitro ubiquitination of E2F1 by multiple ROC-cullin ligases. Cancer res. 61, 1347-1353 (2001) and Maeda, I. Ohta, T. Koizumi, H. Fukuda, M. In vitro ubiquitination of cyclin D1 by ROC1-CUL1 and ROC1-CUL3. FEBS Lett. 494, 181-185 (2001).

Rabbit E1 (Affiniti Research Products), bovine ubiquitin (Sigma) and Flag-ubiquitin (Sigma) were purchased commercially. His-UbcH5c, and truncated N-terminal fragment of His-BRCA1 and His-BARD1 were prepared by Hashizume et al. previously described¹. Full length His-Flag-NPM was obtained by two-step purification using nickel agarose beads followed by anti-Flag cross-linked agarose beads. The purified proteins were stained with Coomassie Brilliant Blue, and their concentrations were determined through a comparison to protein standards using a densitometer (LAS 3000, Fuji film).

### Cell culture, expression and immunological techniques

Cells were cultured in Dulbecco's Modified Eagle's Medium supplemented with 10% fetal calf serum (293T, HeLa and COS7) or 10% newborn calf serum (3T3 Swiss) and 1% antibiotic-antimycotic agent (Life Technologies, Inc). Cells were expressed using the standard calcium phosphate precipitation method as previously described²⁴ in; Ohta, T., Michel, J.J., Schottelius, A. J., & Xiong, Y. ROC1, a homolog of APC11, represents a family of cullin partners with an associated ubiquitin ligase activity. Mol. Cell 3, 535-541 (1999). To analyze the effect of proteasome inhibitors, cells were treated with MG132 (20 µM), LLnL (20 µM) or the same volume of DMSO solvent (1 µl/ml culture) for 10 hours before harvesting. Cell cycle synchronization by thymidine-nocodazole was performed by the method previously described²⁵ in; Ohta, T., Michel, J. J., & Xiong, Y. Association with cullin partners protects ROC proteins from proteasome-dependent degradation. Oncogene 18, 6758-6766 (1999).

The cells were then stained with propidium iodide and subjected to flow cytometry using FACSCalibur (Becton Dickinson).

Immunoprecipitation and immunoblotting methods, including the detection of *in vivo* ubiquitinated substrates, were performed by the method proposed by Hashizume et al. and Nishikawa et al. ^{1,2} except that Flag peptide was used to extract Flag-NPM from anti-Flag antibody-conjugated agarose beads (M2, Sigma) according to the manufacturer's instructions.

The *in vitro* ubiquitination assay was performed by the method proposed by Hashizume et al. and Nishikawa et al.^{1,2} as mentioned above except that His-Flag-NPM (0.5 µg) was added to the reaction.

The *in vitro* kinase assay was performed as reported²⁶ in; Ohta, T., & Xiong, Y. Phosphorylation-and Skp1-independent in vitro ubiquitination of E2F1 by multiple ROC-cullin ligases. Cancer Res. 61, 1347-1353 (2001). For dephosphorylation of immunoprecipitated HA-BARD1, HA-BARD1 immobilized beads were incubated with 2U of calf intestinal alkaline phosphatase (Takara) at 37°C for 30 minutes.

Using anti-CDK2 or anti-HA immunocomplex as a kinase as precipitated from the 293T cells expressing CDK2 cyclin E1 or HA-CDK1 cyclin B1, a kinase assay was performed for the substrate protein (2 µg). For preparation of nucleus fractions and cytoplasm fractions, the cells were dissolved at 4°C for 5 minutes in a buffer A [10 mM/L MgCl₂, 100 mM/L NaCl, 0.5% Triton X-100, 2.5 mM/L MgCl₂, 10 mM/L NaF, and a protenase inhibitor] and passed through three times with a 26-gauge syringe.

Cytoplasm supernatant fractions were obtained after centrifugal separation at 4,000 x g for 2 minutes. In obtain nucleus fractions from the remaining pellets, ultrasonic waves were applied to the buffer B for extraction [50 mM/L Tris-HCl (pH 7.5), 150 mM/L NaCl, 0.5% NP-40, 50 mM/L NaF, 1mM I/L DTT, 1 mM/L NaVO₃, and a protenase inhibitor].

After the 293 cells were labeled with [³⁵S]-methionine for 1 hour for pulse labeling, pulse-chase analysis was monitored.

### Screening using mass spectrometry

The ubiquitination reaction was carried out using Flag-ubiquitin, E1, His-UbcH5c and anti-Myc immunocomplex immobilized on protein A agarose beads precipitated from 293T cells expressing Myc-BRCA1 (1-772) and BARD1 by the same procedures as in the method by Nishikawa et al². The supernatant from ten reactions (30 µl each) was collected and incubated with a 30 µl volume of anti-Flag antibody-cross linked beads (Sigma) to precipitate proteins conjugated with Flag-ubiquitin. The proteins conjugated with Flag-ubiquitin were eluted off the beads in 30 µl of 25 mM ammonium bicarbonate containing 0.1 mg/ml of Flag peptide and digested with 7.4 µg/ml of trypsin for 20 hours at 30°C.

Alternatively, proteins interacting with the complex of HA-BARD1(1-408) and either wild-type or mutant (I26A) Flag-BRCA1(1-222) were immunoprecipitated and extracted as described above, separated by SDS-PAGE, and stained with Sypro Ruby (Molecular Probe) according to the manufacturer's instructions. The target bands were excised from the gel and digested with trypsin using the In Gel Digest Kit (Millipore) according to the manufacturer's instructions.

The peptide fragments were analyzed by LC/MS/MS according to the method by Nishikawa et al. as described above². The acquired collision-induced dissociation spectra were analyzed by Mascot software.

### Indirect immunocytochemistry

Proliferating cells were fixed with 3% formalin for 15 minutes and permeated through 0.2% Triton X-100 for 5 minutes. Cells were washed with phosphate-buffered saline (PBS), blocked with 0.5% BSA in PBS, and stained with indicated antibodies: anti-NPM antibody, anti-α/β tubulin antibody, anti-BARD1 antibody and anti-BRCA1 antibody.

For detecting both BRCA 1 and NPM colocalized at spindle poles, cells were fixed with cold methanol and permeated through 0.1% Triton X-100 buffer as reported¹⁵ in Hsu, L.C, & White RL. BRCA1 is associated with the centrosome during mitosis. Proc. Natl. Acad. Sci. U.S.A. 95, 12983-12988 (1998). Primary antibodies were diluted in the blocking buffer at the following concentrations: anti-NPM (0.5 µg/ml); anti-α/β-tubulin (1 µg/ml); anti-BARD1 (3 µg/ml); and anti-BRCA1 (2 µg/ml). FITC or Rhodamine-conjugated secondary antibodies (Jackson Immunoresearch) were used at 1:50 dilution. The nucleus was counterstained with 0.5 µM TO-PRO-3 (Molecular probe), cells were then mounted with fluorescent mounting medium (BioLad) and examined with a confocal laser scan microscope (LSM 510, Carl Zeiss).

### [Example 1]

### Identification of NPM by two screenings using different substrates ubiquitinated by BRCA1-BARD1 ligase:

First, after hypothesizing that ubiquitin ligase immunocomplexes may contain substrates, we performed the following test. A protein conjugated with Flag-ubiquitin was prepared from the Myc-BRCA1 (1-722)-BARD1 immunocomplexes mentioned above. A reaction supernatant was analyzed by immunoblotting using an anti-Flag antibody (Left panel in Fig. 1a) and the remaining portion was polyubiquitinated and analyzed by nanoscale capillary liquid chromatography-tandem mass spectrometry (LC/MS/MS) (Fig. 1a). Of the proteins identified, nucleophosmin/B23 (NPM) exhibited the highest possibility with 17 peptides displaying a probability based Mowse score of 126 (Table 1).

**Table 1: Peptide segments of NPM identified by screening shown in Fig. 1a and Fig. 1b.**

| a | | b | |
|---|---|---|---|
| 25 | ADKDYHFK (SEQ ID No. 4) | 74 | VTLATLK (SEQ ID No. 21) |
| 33 | VDNDENEHQLSLR (SEQ ID No. 5) | 135 | LLISISGK (SEQ ID No. 22) |
| 46 | TVSLGAGAK (SEQ ID No. 6) | 240 | GPSSVEDIKAK (SEQ ID No. 23) |
| 74 | VTLATLK (SEQ ID No. 7) | 268 | FINYVK (SEQ ID No. 24) |
| 143 | SAPGGGSKVPQKK (SEQ ID No. 8) | | |
| 151 | VPQK (SEQ ID No. 9) | | |
| 198 | DTPAKNAQKSNQNGK (SEQ ID No. 10) | | |
| 203 | NAQKSNQNGKDSK (SEQ ID No. 11) | | |
| 213 | DSKPSSTPRSK (SEQ ID No. 12) | | |
| 216 | PSSTPRSKGQESFK (SEQ ID No. 13) | | |
| 224 | GQESFK (SEQ ID No. 14) | | |
| 230 | KQEK (SEQ ID No. 15) | | |
| 240 | GPSSVEDIK (SEQ ID No. 16) | | |
| 251 | MQASIEKGGSLPK(SEQ ID No. 17) | | |
| 264 | VEAKFINYVK(SEQ ID No. 18) | | |
| 268 | FINYVKNCFR (SEQ ID No. 19) | | |
| 278 | MTDQEAIQDLWQWR (SEQ ID No. 20) | | |

Second, presuming that substrate proteins would associate with a ubiquitin ligase transiently and that this association could be stabilized by the loss of ligase activity, we compared the proteins associated with wild-type BRCA1-BARD1 ligase and catalytically inactive BRCA1(I26A)-BARD1 ligase. Anti-Flag immunocomplex precipitated from the 293T cells expressing one of HA-BARD1 (1-408), wild type Flag-BRCA1 (1-222), or I26A mutant was separated by SDS-PAGE and stained with Sypro Ruby. The protein shifting to near 38-40kDa (arrow) was digested to be provided to LC/MS/MS. The I26A mutation in BRCA1 retains the BARD1 association site, but it disrupts the association with UbcH5c resulting in inactivation of the catalytic activity of the BRCA1-BARD1 heterodimer enzyme⁹. A protein of approximately 38-40 kDa was present only in the mutant ligase complex at a higher level and was identified by LC/MS/MS as NPM (Fig. 1b and Table 1). Thus, two different screening approaches identified the same protein, NPM.

The association between BRCA1-BARD1 and NPM *in vivo* was confirmed using cells expressed transiently by immunoprecipitation (IP)-Western analysis (Fig. 1c). Myc-BRCA1(1-772), HA-BARD1 and Flag-NPM plasmid were combined and expressed simultaneously in the 293T cells. Total cellular solution (2 panels above Fig. 1c) or immunoprecipitation (IP) was provided to immunoblotting (IB) using anti-HA/Myc, anti-HA antibody, and anti-Myc antibody. Anti-HA/Myc represents the immunoblotting method using anti-Ha antibody with subsequent anti-Myc antibody as re-probes. *In vivo* association of NPM with the BRCA1-BARD1 complex was demonstrated by IP-Western blotting analysis (lane 4). If eliminating either BRCA1 or BARD1 from the expression, NPM disappeared. (lanes 1 and 2). This suggests that the association with NPM depends upon BRCA1-BARD1 heterodimer formation.

The 293T cell solution underwent immunoprecipitation with an antibody to BARD1, and was analyzed by Western blotting. As a result, BARD1 (Fig. 1d, lane 1, upper panel) and NPM (Fig. 1d, lane 1, lower panel) were detected in the BARD1 immunocomplex and demonstrated specific competition with the BARD1 antigen peptide (Fig. 1d, lane 2). Thus, endogenous NPM appears to be physically associated with the BRCA1-BARD1 ligase complex and to become its substrate.

### [Example 2]

### Confirmation of NPM ubiquitination by BRCA1-BARD1.

To determine whether NPM is a substrate of the BRCA1-BARD1 ubiquitin ligase, Flag-NPM was co-expressed in 293T cells with HA-tagged ubiquitin, Myc-BRCA1 (1-772) and BARD1 (Fig. 2a). Thirty-six hours after co-expression, cells were recovered and boiled in 1% SDS containing buffer, and after being diluted up to 0.1%, Flag-NPM was immunoprecipitated using anti-Flag antibody. Subsequently, the NPM precipitation separated with SDS-PAGE using anti-HA antibody (Upper part in Fig. 2a) or anti-Flag antibody (lower part in Fig. 2a) was analyzed by the immunoblotting of NPM, which demonstrated a ladder characteristic of polyubiquitinated NPM (lane 3). When removing either Flag-NPM, HA-ubiquitin, Myc-BRCA 1 (1-772), or BARD1, no NPM ladders were detected, which supported the concept of BRCA1-BARD1-dependent NPM ubiquitination.

Another RING type E3 ligase, MDM2, is known to effectively accelerate ubiquitination of a cancer suppressing gene p53 that is a known substrate¹⁰. In a parallel *in vivo* ligase assay, *in vivo* ubiquitinated Myc-p53 and Flag-NPM were detected as mentioned above. (Fig. 2b, lanes 3 and 6). In contrast, BRCA1-BARD1 did not cause noticeable p53 ubiquitination (lane 2).

To eliminate the possibility that NPM ubiquitination was caused by misfolding due to ectopic overexpression, we examined whether endogenous NPM is also ubiquitinated by BRCA1-BARD1 (Fig. 2c). HA-tagged ubiquitin was co-expressed in 293T cells with Myc-BRCA1 (1-772) and BARD1. Endogenous NPM was immunoprecipitated from 293T cells using 1.5 µg pf amto-NPM antibody, and NPM ubiquitination was analyzed by immunoblotting using anti-HA antibody. Polyubiquitinated NPM was readily detected when wild type ligase was added (lane 4). However, NPM ubiquitination disappeared when the I26A mutation was substituted in BRCA1, demonstrating a dependence upon the ubiquitin ligase activity of BRCA1-BARD1 (lane 5).

We further tested whether BRCA1-BARD1 ubiquitinates NPM in a *in vitro* system using a recombinant protein alone (Fig. 2d). Recombinant His-Flag-NPM purified by E. coli was incubated in the presence of ATP with purified ubiquitin, E1, E2/His-UbcH5c, His-BRCA1 (1-304), and His-BARD1 (14-189). After the reaction product was separated by SDS-PAGE, immunoblotting was performed using anti-Flag antibody. As a result, two slowly floating products were detected in the gel (lane 7). When eliminating substrate NPM, ubiquitin, E2/His-UbcH5c, BRCA1 or BARD1, NPM ubiquitination disappeared completely. This demonstrated BRCA1-BARD1-dependent NPM ubiquitination in a purified system. Therefore, we concluded that NPM is a substrate of the BRCA1-BARD1 E3 ubiquitin ligase.

### [Example 3]

### Confirmation of whether NPM ubiquitination by BRCA1-BARD1 can be a signal for proteasomal proteolysis

As mentioned above, the present inventors and others recently discovered that BRCA1-BARD1 catalyzes untraditional Lys-6-linked polyubiquitin chain formations which are deubiquitinated by purified 26S proteasome *in vitro* instead of being targeted for proteolysis^{2, 3}. Therefore, we tested whether BRCA1-BARD1-mediated NPM ubiquitination targets NPM for proteolysis by measuring its stability *in vivo* (Fig. 3). Namely, Flag-NPS along with Myc-BRCA1¹⁻⁷⁷² and HA-BARD1 plasmid was expressed in 293T cells within a 6 well plate. An adjustment was made by adding pcDNA3 vector such that the total plasmid DNA per well became 2.5 µg. The amount of intracellular expression of each protein was analyzed by immunoblotting using anti-HA antibody, anti-Flag antibody, and anti-α/β tubulin antibody. The steady level of expression of Flag-NPM in 293T cells increased in a dose-dependent manner of BRCA1-BARD1 rather than decreasing due to co-expression of BRCA1-BARD1 (Fig. 3a).

Subsequently, 293T cells expressing Flag-NPM, pcDNA3 and Myc-BRCA1/BARD1 were incubated with cyclohexamide (10 µM), and the results were monitored at 4, 8, and 12 hours. The amount of intracellular expression of Flag-NPM was analyzed by immunoblotting using anti-Flag antibody. Pulse-chase analysis also supported that BRCA1-BARD1 stabilizes NPM (Fig. 3b).

Furthermore, from the cells treated with one of the proteasome inhibitors MG132 (20 µM) or LLnL (20 µM) or DMSO solvent for 10 hours, *in vivo* BRCA1-BARD1-mediated ubiquitinated Flag-NPM was detected as described in Embodiment 2. However, the amount of ubiquitinated Flag-NPM did not increase. (Fig. 3c). These findings suggested that BRCA1-BARD1-mediated NPM ubiquitination affects the functioning of NPM through a non-proteolytic mechanism.

### [Example 4]

### Confirmation of colocalization of NPM with BRCA1-BARD1 and NPM ubiquitination in mitosis

A rabbit polyclonal antibody specific to the C-terminal of BARD1 was prepared and used to examine its intracellular localization (subcellular) and its coexistence with NPM during the cell cycle (Fig. 4).

Proliferating Swiss 3T3 cells (a) or COS7 cells (b, c) were fixed respectively with 3% formalin or cold methanol. These cells were stained with anti-NPM antibody, anti-α/β tubulin antibody, anti-BARD1 antibody, and anti-BRCA1 and subsequently with FITC (green) or Rhodamin (red) conjugated secondary antibodies. The nucleus was stained with TO-PRO-3 (blue).

The results revealed that in the interphase cells, BARD1 is localized within nucleoli other than nucleolus, while NPM is localized in the nucleolus, but both did not coexist (Fig. 4a, left three cells in each panel). In contrast, in the mitotic cells, NPM colocalizes with BARD1 particularly around the mitotic spindle (Fig. 4a, a cell at right of each panel). We next examined the localization of BRCA1 and NPM under conditions where BRCA1 and NPM are readily detected at the spindle poles (Fig. 4b). BRCA1 colocalized with NPM at the spindle poles (Fig. 4c). This suggests a cell cycle-dependent BRCA1-BARD1-NPM interactions and functional adjustment of NPM by BRCA1-BARD1 during mitosis.

Furthermore to determine whether mitotic association of BRCA1-BARD1 and NPM is correlated with cell cycle-dependent NPM ubiquitination, we synchronized HeLa cells at the G2/M boundary by a thymidine-nocodazole treatment, subsequent reentry to the cell cycle after 0.5, 1, 2, and 4 hours, followed by a release into a G1 phase (Fig. 4d). Cell cycle synchronization was monitored by flow cytometry. The synchronized cells were boiled in a 1% SDS buffer solution, diluted and underwent immunoprecipitation using 1.5 µg anti-NPM antibody. Finally the cells were analyzed by immunoblotting using anti-ubiquitin antibody. Prior to the immunoblotting, a nitrocellulose film was boiled to increase recognition sensitivity. Thus, *in vivo* NPM ubiquitination was assessed by IP-Western analysis. NPM ubiquitination was detected immediately after the cells were released from the mitotic block (Points at 0.5 hour and 1 hour), but was not seen in mitotically arrested cells or in the G 1 phase cells.

### [Example 5]

### Investigation of phosphorylation using CDK 1/2 at the NH₂ terminal of BARD 1

We next attempted to determine the changes in the molecular weight of BARD1 due to the co-expression of CDK/cyclin in order to investigate whether BARD 1 is phosphorylated by CDK.

Namely, NH₂ terminal (1-320) fragments or COOH terminal (411-777) fragments of Myc-BRCA1¹⁻⁷⁷² and HA-BARD1 were co-expressed with CDK-cyclin or pcDNA3 vector in 293T cells. When HA-BARD1⁴¹¹⁻⁷⁷⁷ (COOH terminal fragments 411-777 of BARD1) was co-expressed with CDK/cyclin, no changes were found. However, when HA-BARD1¹⁻³²⁰ (NH2 terminal fragments 1-320 of BARD1) was co-expressed with CDK/cyclin, a moderate movement of proteins were observed in the gel (Fig. 6 A). When HA-BARD1¹⁻³²⁰ was analyzed by immunoblotting, at least three products were obtained (Fig. 6 A, B, and D, arrows) Subsequently, HA-BARD1¹⁻³²⁰ underwent immunoprecipitation and was analyzed by immunoblotting using anti-HA antibody. HA-BARD1¹⁻³²⁰ fixed with agarose beads were incubated with alkali phosphatase (AP+) or in a buffer alone (-). As a result, when treated with alkali phosphatase, the aforementioned three products disappeared (Fig. 6 C). Therefore, these three products were determined to be phosphorylated compounds of BARD1. Next, in order to investigate whether the said kinase directly phosphorylates BARD1, the inventors investigated whether the purified recombinant NH₂ terminal fragments can be a substrate of CDK *in vitro.* Purified recombinant GST (Fig. 6 and Fig. 7D, lanes 1, 4, and 7), His-BARD1¹⁴⁻¹⁸⁹ (Lanes 2, 5, and 8), and His-BARD1¹⁻³²⁰ (Lanes 3, 6, and 9) were separated by SDS-PAGE, and then, stained by Coomassie Brilliant Blue (CBB, left) or incubated along with [γ-³²P]ATP and CDK2-cyclin E1 (Lanes 4-6) or CDK1-cyclin B1 (Lanes 7-9). Proteins were then separated by SDS-PAGE and analyzed by autoradiography.

As a result, both His-BARD¹⁴⁻¹⁸⁰ and HA-BARD1¹⁻³²⁰ were phosphorylated *in vitro* with CDK2-cyclin E1 and CDK1-cyclinc B1 (Fig. 6 C).

As in the results of *in vivo* movement shift of HA-BARD1¹⁻³²⁰, *in vitro* phosphorylated HA-BARD1¹⁻³²⁰ indicated several bands and demonstrated one or more phosphorylation sites in the HA-BARD1¹⁻³²⁰ fragments. This means that the ability of *in vitro* phosphorylation of purified BARD1 is not particularly needed for BRCA1 to have interactions with the said kinase. The fact that the co-expression of BRCA1 does not have any effect on the amount of phosphorylation of BARD1 supports this concept. The inventors performed mapping of four phosphrylation sites in BARD1 by mutation analysis and identified mutants of BARD1S148A/S251A/S288A/T299A.

These showed almost no changes in molecular weight when using CDK2-cyclin E1 (Fig. 6D) or CDK1-cyclin B1. Based on these results, BARD1 was found to be phosphorylated by CDK1 and CDK2 at the NH₂ terminals in an independent mode from the heterodimer formation of BRCA1-BARD1.

### [Example 6]

### Understanding of the molecular mechanism that is a cause for NPM ubiquitination by BRCA1-BARD1 depending on cell cycle

The inventors attempted to understand the molecular mechanism that may be the cause for the NPM ubiquitination by BRCA1-BARD1 only during the mitosis phase. It has been reported that NPM is phosphorylated by CDK2-cyclin E at the G1/S transition phase resulting in dissociation of NPM from centrosomes to allow daughter centrioles to divide^{7,8}.

NPM is prevented from associating with the centrosomes during S phase while daughter centrioles mature into centrosomes and, subsequently, into two spindle poles. Since NPM is regulated by phosphorylation of CDK2-cyclin during centrosome replication, we investigated the possibility that CDK2 may affect NPM ubiquitination by the BRCA1-BARD1 ligase. Myc-BRCA1 (1-772), BARD1, Flag-NPM and HA-tagged ubiquitin were expressed in 293 T cells. The *in vivo* ubiquitinated products by BRCA1-BARD1 were detected as in Embodiment 2. CDK-cyclin was also re-expressed. To our surprise, both CDK2-cyclin E and CDK2-cyclin A completely inhibited NPM ubiquitination *in vivo* (Fig. 7A, lanes 3 and 4). In the same assay, CDK1-cyclin B could not interfere with BRCA1-BARD1-mediated NPM ubiquitination (Lane 5). This suggested that CDK2 has a specific function in inhibiting the BRCA1-BARD1 ligase activity.

### [Example 7]

### Inhibition of BRCA1-BARD1 ubiquitin ligase activity by CDK2

In order to distinguish these two models, the inventors tested whether BRCA1-BARD1 ligase can catalyze ubiquitination of NPM (T199A) which is a mutant having no CDK2 phosphorylation sites⁸ (Fig. 7B). The procedures were the same as those described in Embodiment 6. CDK-cyclin was co-expressed and a Flag-NPM phosphorylation site mutant was used instead of a wild type. NPM (T199A) was ubiquitinated by BRCA1-BARD 1 ligase (Lane 1), and the ubiquitination was inhibited by CDK2 (Lane 2).

Therefore, it was suggested that an inhibition of NPM ubiquitination by CDK2 is not attributed to phosphorylation of NPM.

Next, the inventors tested the effect of CDK2-cyclin E and CDK2-cyclin A on the intrinsic ligase activity of the BRCA1-BARD1 heterodimeric complex by measuring BRCA1 autoubiquitination. The procedures were the same as those described in Embodiment 6. An anti-Myc antibody was used in the immunoprecipitation for detecting Myc-BRCA1, which was autoubiquitinated with the co-expression of CDK-cyclin. In contrast to the fact that CDK1-cyclin B did not inhibit the autoubiquitination of BRCA1, both CDK2-cyclin E and CDK2-cyclin A completely inhibited autoubiquitination of BRCA1 (Fig. 7C). The aforementioned results revealed that both CDK2-cyclin E and CDK2-cyclin A catalyze phosphorylation of BARD1 and they can also inhibit BRCA1-BARD 1 ligase activity to the substrate including NPM.

Since BARD1 is the substrate of CDK2-cyclin, inhibition of the BRCA1-BARD1 ubiquitin ligase activity may have a direct influence on phosphorylation of BARD1. However, BARD1 is a non-phosphorylation mutant so that autoubiquitination of BRCA1 via BARD1 S148A, S288A/T299 can be inhibited by CDK2-cyclin E1 (Fig. 7D).

The aforementioned non-phosphorylation mutant of BARD1 remains to have a sensitivity to the inhibition of ubiquitin ligase activity by CDK2. Although CDK1 phosphorylates BARD1 at the same site as that for CDK2, BRCA1-BARD1 ligase activity is not inhibited so that it appears that phosphorylation itself is not so important in the downregulation of the BRCA1-BARD1 ligase.

### [Example 8]

### Investigation of the effects of CDK2 on the levels of expression of proteins of BRCA1 and BARD1

The inventors investigated how CDK2 influences the levels of expression of proteins of BRCA1 and BARD1. Namely, Myc-BRCA1¹⁻⁷⁷² and HA-BARD1 were expressed in 293T cells and followed by immunoblotting using an anti-HA antibody then subsequently an anti-Myc antibody as a re-probe.

In response to the ubiquitin ligase activity, the steady state of both levels in BRCA1 and BARD1 severely decreased by CDK2-cyclin E1/A1 (Fig. 8A, lanes 3 and 4) rather than by CDK1-cyclin B1 (Fig. 8A lane 5). In a 60 mm dish containing 293T cells, CDK2-cyclin E1 was placed in an amount of 0.1, 0.5, and 1.5 µg, and various plasmids (each 1 µg) for coding Myc-BRCA1¹⁻⁷⁷² and HA-BARD1 were expressed, and steady states of various proteins were analyzed by immunoblotting using an anti-HA antibody, an anti-Flag antibody, and an anti-Myc antibody. The results demonstrated that the steady state of both levels in BRCA1 and BARD1 decreased in a dose-dependent manner (Fig. 8B). There was no variance in stability in BRCA1- BARD1 with the presence of CDK1-cyclin. This was not due to the absence of kinase activity in the cells because the phosphorylation of BARD1 was detected clearly (Fig. 8A, lane 5).

The 293T cells expressing Myc-BRCA1¹⁻⁷⁷², HA-BARD1 and parent pc DNA3 vector (upper column) or CDK2 cyclin E1 (lower column) were supplemented with [³⁵S] for performing a pulse chase test. Subsequently, the cellular solution was treated with an anti-Myc antibody for immunoprecipitation and separated by SDS•PAGE. The results were analyzed by autoradiography. The colocalization of CDK2-cyclin E1 noticeably reduced the half life of the BRCA1-BARD1 complex in the cells (up until 2 hours or less between 4 and 6 o'clock: Fig. 8C). This decrease in expression was assumed to be due to proteolysis.

### [Example 9]

BRCA 1 and BARD 1 were reported to be decomposed during the transportation from the nucleus to cytoplasm. The inventors investigated how CDK2-cyclin E1 has an effect on localization with BRCA1 when both CDK2-cyclin E1 and BRCA1 were co-expressed. Flag-BRCA1, HA-BARD1, and parent pcDNA3 vector or CDK2cyclin E1 were transiently expressed on 293T cells. In order to prevent the degradation of cytoplasmic BRCA1, carbobenzoxyl-leucinyl-leucinyl-leucinal (MG132), which is a proteasome inhibitor, was added to the cells 6 hours prior to the time for recovering cells. For quantitative analysis of the BRCA1 protein levels in the nucleus and cytoplasm, cells were fractionated into a nucleus (N) pool and a cytoplasm (C) pool and immunoblotting was performed. When CDK2-cyclin E1 was not present, the cytoplasmic BRCA1 protein level was much lower than the protein level found in the nucleus (Fig. 8D, upper column, lanes 1 and 2). In the co-expression of CDK2-cyclin E1, the cytoplasmic BRCA1 protein level exceeded the protein level found in the nucleus (Fig. 8D, 2^{nd} column from the top, lanes 3 and 4). According to the results mentioned above, it was assumed that due to co-expression of CDK2-cyclin E1, BRCA1 is transported from the nucleus to the cytoplasm.

### [Example 10]

### Expression of BARD 1 dependent upon the cell cycle

The following experiment was carried out in order to investigate how BARD1 is expressed in the cell cycle. HeLa cells were synchronized by performing a double thymidine inhibition (A) to the G₁/S transition phase. Also, the cells were synchronized to the mitotic phase by a performing thymidine-nocodazole inhibition (B). After releasing the inhibition, the courses of the cell cycle were monitored by FACS analysis. The cells were also analyzed by immunoblotting using antibodies at an appropriate time after releasing inhibition. The results demonstrated that the level of expression of BARD1 was highest during the mitotic phase, showing a mild movement band (Fig. 5A lane 8, b lane 1~3). It agreed with the enhanced expression of BRCA1 and cyclin B1. BARD1 was divided into two bands at the M/G₁ transition phase (Fig. 5A, lane 9, b lane 4).
They shifted to dominant rapid bands (Fig. 5A lane 10, b lane 5). Synchronous to these shifts, BRCA1 and cyclin B1 disappeared. The expression level of BARD1 was subject to downregulation at G₁ phase and S phase, and in particular, noticeable downregulation was detected at G₁ late phase when cyclin E1 was expressed (Fig. 5A lane 2, b lane 8).

The following references are incorporated in the entire specification as references.

### References

1. Hashizume, R. et al. The RING heterodimer BRCA1-BARD1 is a ubiquitin ligase inactivated by a breast cancer-derived mutation. J. Biol. Chem. 276, 14537-14540 (2001).
2. Nishikawa, H. et al. Mass spectrometric and mutational analyses reveal Lys-6-linked polyubiquitin chains catalyzed by BRCA1-BARD1 ubiquitin ligase. J. Biol. Chem. in press [online resource] http://www.jbc.org/cgi/reprint/M308540200v1 (2003).
3. Wu Baer, F., Lagrazon, K., Yuan, W., & Baer, R. The BRCA1/BARD1 heterodimer assembles polyubiquitin chains through an unconventional linkage involving lysine residue K6 of ubiquitin. J. Biol. Chem. 278, 34743-34746 (2003).
4. Baer, R., & Ludwig, T. The BRCA1/BARD1 heterodimer, a tumor suppressor complex with ubiquitin E3 ligase activity. Curr. Opin. Genet. Dev. 12, 86-91 (2002).
5. Venkitaraman, A. R. Cancer susceptibility and the functions of BRCA1 and BRCA2. Cell 108, 171-182 (2002).
6. Deng, C. X. Roles of BRCA1 in centrosome duplication. Oncogene 21, 6222-6227 (2002).
7. Okuda, M. et al. Nucleophosmin/B23 is a target of CDK2/cyclin E in centrosome duplication. Cell 103, 127-140 (2000).
8. Tokuyama, Y. et al. Specific phosphorylation of nucleophosmin on Thr(199) by cyclin-dependent kinase 2-cyclin E and its role in centrosome duplication. J. Biol. Chem. 276, 21529-21537 (2001).
9. Brzovic, P. S. et al. Binding and recognition in the assembly of an active BRCA1/BARD1 ubiquitin-ligase complex. Proc. Natl. Acad. Sci. U.S.A. 100, 5646-5651 (2003).
10. Honda, R., Tanaka, H., & Yasuda, H. Oncoprotein MDM2 is a ubiquitin ligase E3 for tumor suppressor p53. FEBS Lett. 420, 25-27 (1997).
11. Hershko, A., & Ciechanover, A. The ubiquitin system. Annu. Rev. Biochem. 67, 425-479 (1998).
12. Pickart, C. M. Ubiquitin enters the new millennium. Mol. Cell 8, 499-504 (2001).
13. Xu, X. et al. Centrosome amplification and a defective G2-M cell cycle checkpoint induce genetic instability in BRCA1 exon 11 isoform-deficient cells. Mol. Cell 3, 389-395 (1999).
14. Weaver, Z. et al. Mammary tumors in mice conditionally mutant for Brcal exhibit gross genomic instability and centrosome amplification yet display a recurring distribution of genomic imbalances that is similar to human breast cancer. Oncogene 21, 5097-5107 (2002).
15. Hsu, L. C, & White RL. BRCA 1 is associated with the centrosome during mitosis. Proc. Natl. Acad. Sci. U S.A. 95, 12983-12988 (1998).
16. Hsu, L. C., Doan, T. P., & White, R. L. Identification of a gamma-tubulin-binding domain in BRCA1. Cancer Res. 61, 7713-7718 (2001).
17. Zatsepina, O. V. et al. The nucleolar phosphoprotein B23 redistributes in part to the spindle poles during mitosis. J. Cell. Sci. 112, 455-466 (1999).
18. Wu, M. H. & Yung, B. Y. UV stimulation of nucleophosmin/B23 expression is an immediate-early gene response induced by damaged DNA. J. Biol. Chem. 277, 48234-48240 (2002).
19. Tawfic, S., Olson, M. O., & Ahmed, K. Role of protein phosphorylation in posttranslational regulation of protein B23 during programmed cell death in the prostate gland. J. Biol. Chem. 270, 21009-21015 (1995).
20. Pang, Q. et al. Nucleophosmin interacts with and inhibits the catalytic function of eukaryotic initiation factor 2 kinase PKR. J Biol. Chem. 278, 41709-41717 (2003).
21. Okuwaki, M., Iwamatsu, A., Tsujimoto, M., & Nagata, K. Identification of nucleophosmin/B23, an acidic nucleolar protein, as a stimulatory factor for in vitro replication of adenovirus DNA complexed with viral basic core proteins. J. Mol. Biol. 311, 41-55 (2001).
22. Okuwaki, M., Matsumoto, K., Tsujimoto, M., & Nagata, K. Function of nucleophosmin/B23, a nucleolar acidic protein, as a histone chaperone. FEBS Lett. 506, 272-276 (2001).
23. Keyomarsi, K. et al. Cyclin E and survival in patients with breast cancer. N. Engl. J. Med. 347, 1566-1575 (2002).
24. Ohta, T., Michel, J. J., Schottelius, A. J., & Xiong, Y. ROC1, a homolog of APC11, represents a family of cullin partners with an associated ubiquitin ligase activity. Mol. Cell 3, 535-541 (1999).
25. Ohta, T., Michel, J. J., & Xiong, Y. Association with cullin partners protects ROC proteins from proteasome-dependent degradation. Oncogene 18, 6758-6766 (1999).
26. Ohta, T., & Xiong, Y. Phosphorylation- and Skp1-independent in vitro ubiquitination of E2F1 by multiple ROC-cullin ligases. Cancer Res. 61, 1347-1353 (2001).
27. Maeda, I. Ohta, T. Koizumi, H. Fukuda, M. In vitro ubiquitination of cyclin D1 by ROC1-CUL1 and ROC1-CUL3. FEBS Lett.494, 181-185(2001).

### Sequence listing free text

SEQ ID No. 3: Synthetic peptide

## Claims

1. A method of polyubiqutinating a nucleophosmin comprising of reacting the nucleophosmin with BRCA1-BARD1.

2. A method of stabilizing a nucleophosmin comprising of polyubiqutination of nucleophosmin.

3. The method of Claim 1 or 2, wherein polyubiquitination is carried out *in vitro* or *in vivo*.

4. A method of inhibiting polyubiquitination of nucleophosmin comprising of phosphorylating BARD1 using CDK2-cyclin E or CDK2-cyclin A.

5. A method of degrading and/or dissociating BRCA1-BARD1 comprising of phosphorylating BARD1 using CDK2-cyclin E and/or CDK2-cyclin A.

6. A method of inactivating ubiquitin ligase activity of a BRCA1-BARD1 comprising of phosphorylating BARD1 using CDK2-cyclin E and/or CDK2-cyclin A.

7. The method according to any one of Claims 4 to 6 wherein the phosphorylation sites of BARD1 are at least three sites selected from the group consisting of S148, S251, S288 and T299.

8. The method according to any one of Claims 4 to 6 wherein the phosphorylation sites of BARD 1 are S 148, S288 and T299.

9. The method according to any one of Claims 4 to 6 wherein the phosphorylation sites of BARD1 are S148, S251, S288 and T299.

10. A method of transporting BRCA1 from a nucleus to cytoplasm wherein BRCA1 and CDK2-cyclin E and/or CDK2-cyclin A are co-expressed.
